# EUROPÄISCHE PATENTSCHRIFT

(11) **EP 1 149 070 B1**
(45) Veröffentlichungstag und Bekanntmachung des Hinweises auf die Patenterteilung: **25.06.2003**
(21) Anmeldenummer: 00903582.5
(22) Anmeldetag: 17.01.2000
(51) Int. Cl.: C07C 281/02

(54) **VERFAHREN ZUR HERSTELLUNG VON METHYLCARBAZAT**
METHOD FOR PREPARING METHYL CARBAZATE
PROCEDE POUR PREPARER DU CARBAZATE DE METHYLE

(30) Priorität: 26.01.1999 DE 19902960
(43) Veröffentlichungstag der Anmeldung: 31.10.2001
(73) Patentinhaber: Bayer Aktiengesellschaft, 51368 Leverkusen (DE)
(72) Erfinder: RODEFELD, Lars, D-51375 Leverkusen (DE); BEHRE, Horst, D-51519 Odenthal (DE); KLAUSENER, Alexander, D-50259 Pulheim (DE); SÖLLNER, Robert, D-51373 Leverkusen (DE)
(86) Internationale Anmeldenummer: EP0000310
(87) Internationale Veröffentlichungsnummer: WO00044708

(56) Entgegenhaltungen:
- EP-A- 0 103 400
- DE-A- 3 443 820
- DE-C- 285 800
- DATABASE WPI Week 197438 Derwent Publications Ltd., London, GB; AN 1974-67396v XP002133241 & SU 407 888 A (BIOLOG MEDIC CHEM INST), 25. April 1974 (1974-04-25)

## Beschreibung

Die vorliegende Erfindung betrifft ein besonders vorteilhaftes Verfahren zur Herstellung von Methylcarbazat durch Umsetzung von Hydrazin mit Dimethylcarbonat.

Methylcarbazat, das man auch als Hydrazinocarbonsäuremethylester bezeichnen kann, ist ein vielfach verwendbarer Synthesebaustein der organischen Chemie, der z.B. zur Übertragung von Hydrazin oder als Schutzgruppe für Carbonyle dienen kann (s. z.B. Technical Information "Methyl carbazate" der Bayer AG 4.97) und die dort angeführte Literatur.

Die Synthese von Methylcarbazat aus Hydrazinhydrat und Dimethylcarbonat ist bekannt (s. O. Diels, Ber. **1915**, 2183-2195). Das allgemeine Formelschema dazu ist in Gleichung (1) angegeben.

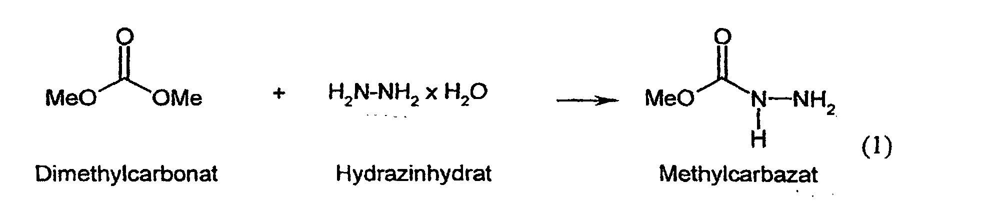

Die Durchführung des Verfahrens erfolgt so, daß man die Komponenten nacheinander zusammenfügt und die Reaktion unter starker Selbsterwärmung ablaufen läßt. Danach werden flüchtige Bestandteile durch Destillation bei 40 mbar und bei einer Badtemperatur von 70°C entfernt.

DE 285 800 beschreibt ein sehr ähnliches Verfahren zur Herstellung von Methylcarbazat.

EP-A 0 103 400 beschreibt ein Verfahren, bei dem man ebenfalls die Komponenten nacheinander zusammenfügt und dabei Dimethylcarbonat in leichtem Überfluß einsetzt. Die Reaktion wird zunächst bei 50°C, später bei 25°C durchgeführt. Die destillative Entfernung von Wasser und Methanol erfolgt durch Destillation bei 40 mbar.

DE 34 43 820 beschreibt schließlich die Herstellung von Methylcarbazat, indem Dimethylcarbonat vorgelegt und Hydrazinhydrat sehr schnell hinzudosiert wird. Dabei erhitzt sich der Reaktionsansatz sehr stark. Flüchtige Bestandteile werden anschließend im Vakuum entfernt.

Führt man die Synthese von Methylcarbazat nach Diels, DE 285 800 oder EP-A 0 103 400 in technischem Maßstab durch, so erhält man stets Produkte, die durch die unerwünschten Komponenten der Formeln (I) und (II) bis zu jeweils ca. 3 Gew.-% verunreinigt sind.

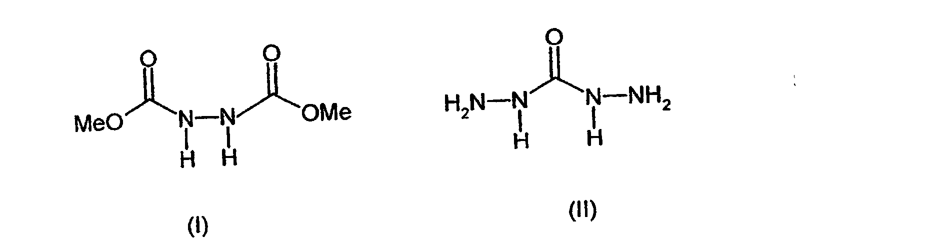

Die in DE 34 43 820 beschriebene Methode führt zu Produkten, in denen die unerwünschte Komponente der Formel (I) besonders stark angereichert ist, z.B. in einer Menge bis zu 4 Gew.-%.

Zudem liefern alle oben beschriebenen Verfahren Methylcarbazat, das sich nach einigen Tagen rötlich verfärbt. Dies ist nachteilig, weil Methylcarbazat zur Herstellung von pharmazeutischen Produkten herangezogen wird, wobei verfärbte Produkte nicht erwünscht sind.

Außerdem enthält nach allen obigen Verfahren hergestelltes Methylcarbazat einen Restgehalt an kanzerogenem Hydrazin von etwa 100 bis 500 ppm und einen Restgehalt an Wasser von im allgemeinen mehr als 1 Gew.-%. Diese Restgehalte sind in der angewendeten destillativen Aufarbeitung nicht weiter zu erniedrigen. Problematisch ist insbesondere der hohe Restgehalt an Hydrazin, der bei der Handhabung solcher Produkte besonderen arbeitshygienischen Aufwand erfordert.

Es besteht deshalb immer noch das Bedürfnis nach einem Verfahren zur Herstellung von Methylcarbazat, bei dem ein Produkt anfällt, das verringerte Anteile an den unerwünschten Komponenten der Formeln (I) und (II), an restlichem Hydrazin und an restlichem Wasser aufweist und außerdem weniger zur Verfärbung neigt als nach bekannten Verfahren hergestelltes Methylcarbazat.

Es wurde nun ein Verfahren zur Herstellung von Methylcarbazat aus Hydrazin und Dimethylcarbonat gefunden, das dadurch gekennzeichnet ist, daß man Hydrazin und Dimethylcarbonat simultan bei -20 bis +30°C in ein vorgelegtes erstes Lösungsmittel eindosiert, danach das Lösungsmittel und Leichtsieder bei vermindertem Druck abdestilliert, und dem dann vorliegenden rohen Methylcarbazat entweder ein zweites Lösungsmittel zufügt und dieses dann bei vermindertem Druck abdestilliert oder ein Gas durch es hindurch leitet.

In das erfindungsgemäße Verfahren kann man Hydrazin und Dimethylcarbonat beispielsweise in einem molaren Verhältnis von 0,9 bis 1,1:1 einsetzen. Vorzugsweise beträgt dieses Verhältnis 0,95 bis 1,05:1, insbesondere 1:1.

Das Hydrazin kann man beispielsweise in reiner Form oder als Gemisch mit Wasser einsetzen. Solche Hydrazin/Wasser-Gemische können z.B. 10 bis 85 Gew.-% Hydrazin enthalten. Geeignet ist insbesondere sog. Hydrazinhydrat (64 Gew.-% Hydrazin enthaltend).

Dimethylcarbonat kann in handelsüblicher Form eingesetzt werden.

Die simultane Dosierung von Hydrazin und Dimethylcarbonat erfolgt vorzugsweise bei 0 bis +20°C, insbesondere bei +5 bis +10°C. Unter dem Begriff "simultan dosieren" bzw. "simultane Dosierung" wird dabei nicht nur die Zudosierung exakt gleicher molarer Mengen der beiden Reaktionspartner pro Zeiteinheit verstanden, sondern auch Arbeitsweisen, bei denen pro Zeiteinheit einer der Reaktionspartner in einem molaren Überschuß von nicht mehr als 20 %, vorzugsweise nicht mehr als 5 % (bezogen auf den anderen Reaktionspartner) zudosiert wird.

Als erstes Lösungsmittel kommt beispielsweise Wasser oder ein organisches Lösungsmittel infrage, wobei es sich bei dem organischen Lösungsmittel beispielsweise um geradkettige, verzweigte oder cyclische, unsubstituierte oder substituierte Aliphaten mit 1 bis 20 C-Atomen oder um unsubstituierte oder substituierte Aromaten mit 6 bis 10 C-Atomen handeln kann. Als Substituenten für die Aliphaten und Aromaten kommen z.B. Halogenatome, insbesondere Chloratome, Hydroxygruppen, Gruppen der Formel X-R³ mit X = Sauerstoff oder Schwefel und R³ = Wasserstoff oder geradkettiges oder verzeigtes C₁-C₄-Alkyl, Gruppen der Formel COOR³ mit R³ wie zuvor angegeben und/oder Gruppen der Formel NR³R⁴ mit R³ wie zuvor angegeben und R⁴ unabhängig von R³ von gleichem Bedeutungsumfang wie R³. Von derartigen Substituenten können auch mehrere gleiche oder verschiedene vorhanden sein, beispielsweise bis zu 4 Stück pro Molekül. Infrage kommen als erstes Lösungsmittel auch Hydrazin und Dimethylcarbonat innerhalb der oben angegebenen Begrenzungen für den molaren Überschuß eines dieser Reaktionspartner. Man kann auch beliebige Mischungen der genannten Lösungsmittel einsetzen. Bevorzugt als erstes Lösungsmittel sind geradkettige und verzweigte aliphatische Alkohole mit 1 bis 8 C-Atomen.

Nach Beendigung der Dosierung kann gegebenenfalls eine Nachreaktionszeit eingehalten werden, beispielsweise ein 30- bis 300-minütiges Rühren des Reaktionsgemisches bei +30 bis +60°C, insbesondere bei +40 bis +55°C.

Nach Beendigung der Dosierung und Ablauf der gegebenenfalls eingehaltenen Nachreaktionszeit werden das eingesetzte Lösungsmittel und Leichtsieder, im wesentlichen sind dies gebildetes Methanol, gegebenenfalls mit dem Hydrazin eingebrachtes Wasser und gegebenenfalls vorliegende Überschüsse an Hydrazin oder Dimethylcarbonat, bei vermindertem Druck abdestilliert. Beispielsweise kann man dabei den Druck auf 0,1 bis 30 mbar erniedrigen. Bevorzugt sind Druckerniedrigungen auf 2 bis 20 mbar, insbesondere 5 bis 10 mbar.

Man kann diese Destillation gegebenenfalls 2-stufig durchführen und das bei der Destillation anfallende erste Lösungsmittel ganz oder teilweise für den nächsten Ansatz wiederverwenden.

Auf diese Weise erhält man als Destillationsrückstand ein rohes Methylcarbazat. Dieses wird nun weiter gereinigt, wobei man 2 verschiedene Methoden anwenden kann.

Methode A: Dem rohen Methylcarbazat wird ein zweites Lösungsmittel zugesetzt, bei dem es sich beispielsweise um ein organisches Lösungsmittel handeln kann, wie es oben bei den ersten Lösungsmitteln beschrieben ist. Bevorzugt als zweites Lösungsmittel sind unsubstituierte und substituierte Aromaten wie Benzol, Toluol, Xylol und Chlorbenzol. Das zweite Lösungsmittel wird dann beispielsweise bei Drucken wie für die Destillation des ersten Lösungsmittels beschrieben abdestilliert.

Methode B: Durch das rohe Methylcarbazat wird ein Gas hindurch geleitet. Als Gase kommen z.B. Stickstoff, Luft, Sauerstoff oder Edelgase infrage. Das rohe Methylcarbazat wird dabei zweckmäßigerweise auf einer Temperatur gehalten, bei der es flüssig ist und noch keinen zu hohen Dampfdruck aufweist, beispielsweise bei 75 bis 120°C.

Nach Durchführung des erfindungsgemäßen Verfahrens erhält man als Rückstand ein Methylcarbazat, das, im Vergleich zu nach bisher bekannten Verfahren erhältlichem Methylcarbazat, wesentlich weniger der unerwünschten Komponenten der Formeln (I) und (II), wesentlich weniger Hydrazin und wesentlich weniger Wasser enthält und außerdem wesentlich weniger zur Verfärbung neigt. Die Erzielung dieser Vorteile ist außerordendich überraschend, denn die Änderung der Zudosierung der Reaktanten, die Erniedrigung der anfanglichen Reaktionstemperatur und die ausschließlich auf physikalischen Mehoden beruhenden weitere Reinigung, die im Prinzip auch schon bisher durchgeführt wurde, ließ dies nicht erwarten.

### Beispiele

Soweit nicht anders vermerkt, sind Prozentangaben Gewichtsprozente.

### Beispiel 1

Es wurden 2 800 g Methanol vorgelegt und auf +5°C abgekühlt. Dazu wurden innerhalb von 10 Stunden 7 560 g Dimethylcarbonat und 4 200 g Hydrazinhydrat exakt simultan dosiert, wobei die Temperatur zwischen +5 und +10°C gehalten wurde. Danach wurde auf 50°C erwärmt und diese Temperatur eine Stunde aufrechterhalten. Anschließend wurden zunächst bei dieser Temperatur und sinkendem Druck bis zu 140 bar 5 124 g eines Gemisches enthaltend im wesentlichen Methanol und Dimethylcarbonat abdestilliert, dann bei 80°C und sinkendem Druck bis zu 8 mbar 2 115 g eines Methanol, Wasser und restliches Hydrazin enthaltenden Gemisches. Nun wurde die Apparatur mit Stickstofff belüftet und 1 823 g Toluol zugegeben, welches dann bei 80°C und sinkendem Druck bis zu 8 mbar in Form von 2 561 g eines Toluol, Wasser, Hydrazin und Methylcarbazat enthaltenden Gemisches abdestilliert wurde. Das dabei im Destillationsbogen sich niederschlagende Sublimat wurde bei 75°C abgeschmolzen und in den Reaktor zurücklaufen gelassen.

Im Reaktionsgefäß wurden 6 583 g Methylcarbazat mit einer Reinheit von 98,2 % crhalten. Die unerwünschte Komponente der Formel (I) war zu 1,2 %, die unerwünschte Komponente der Formel (II) zu 0,5 % enthalten. Das Produkt enthielt 0,1 % Wasser und 55 ppm Hydrazin.

### Beispiel 2

Eine Probe des gemäß Beispiels 1 hergestellten Methylcarbazats und eine Probe gemäß der DE 34 43 820 hergestelltes Methylcarbazat wurden nach 1-monatiger Lagerzeit bei +20°C und im Dunkeln hinsichtlich der aufgetretenen Verfärbung analysiert. Dabei wurden Transmissionsgrade und Hazen-Farbzahlen in einer 5 %igen wäßrigen Lösung gemäß DIN 55945 bestimmt. Ergebnisse sind aus der folgenden Tabelle ersichtlich.

**Tabelle**

| | Transmissionsgrade | | | Hazen-Zahl |
|---|---|---|---|---|
| | Tₓ | T_{y} | T_{z} | |
| Methylcarbazat erhalten Gemäß Beispiel 1 | 99,6 | 99,3 | 98,3 | 5 |
| Methylcarbazat erhalten gemäß DE 34 43 820 | 79,1 | 71,0 | 58,6 | 180 |

## Patentansprüche

1. Verfahren zur Herstellung von Methylcarbazat aus Hydrazin und Dimethylcarbonat, **dadurch gekennzeichnet, daß** man Hydrazin und Dimethylcarbonat simultan bei -20 bis +30°C in ein vorgelegtes erstes Lösungsmittel eindosiert, danach das Lösungsmittel und Leichtsieder bei vermindertem Druck abdestilliert, danach dem vorliegenden rohen Methylcarbazat entweder ein zweites Lösungsmittel zufügt und dieses bei vermindertem Druck abdestilliert oder ein Gas durch es hindurch leitet.

2. Verfahren nach Anspruch 1, **dadurch gekennzeichnet, daß** man Hydrazin und Dimethylcarbonat in einem molaren Verhältnis von 0,9 bis 1,1:1 einsetzt.

3. Verfahren nach Ansprüchen 1 und 2, **dadurch gekennzeichnet, daß** man als erstes Lösungsmittel Wasser oder ein organisches Lösungsmittel einsetzt, bei dem es sich um geradkettige, verzweigte oder cyclische, unsubstituierte oder substituierte Aliphaten mit 1 bis 20 C-Atomen, um unsubstituierte oder substituierte Aromaten mit 6 bis 10 C-Atomen um Hydrazin oder um Dimethylcarbonat handelt, die letzten beiden innerhalb der in Anspruch 5 genannten Mengenbegrenzung.

4. Verfahren nach Ansprüchen 1 bis 3, **dadurch gekennzeichnet, daß** man als erstes Lösungsmittel einen geradkettigen oder verzweigten aliphatischen Alkohol mit 1 bis 8 C-Atomen einsetzt.

5. Verfahren nach Ansprüchen 1 bis 4, **dadurch gekennzeichnet, daß** man die simultane Dosierung so durchführt, daß pro Zeiteinheit einer der Reaktionspartner in einem molaren Überschuß von nicht mehr als 20 % (bezogen auf den anderen Reaktionspartner) zudosiert wird.

6. Verfahren nach Ansprüchen 1 bis 5, **dadurch gekennzeichnet, daß** man nach der Dosierung eine Nachreaktionszeit einhält, bei der man bei +30 bis +60°C 30 bis 300 Minuten rührt.

7. Verfahren nach Ansprüchen 1 bis 6, **dadurch gekennzeichnet, daß** man als zweites Lösungsmittel ein organisches Lösungsmittel einsetzt, bei dem es sich um geradkettige, verzweigte oder cyclische, unsubstituierte oder substutierte Aliphaten mit 1 bis 20 C-Atomen, um unsubstutierte oder substituierte Aromaten mit 6 bis 10 C-Atomen, um Hydrazin oder um Dimethylcarbonat handelt.

8. Verfahren nach Ansprüchen 1 bis 7, **dadurch gekennzeichnet, daß** man die Destillationen unter Erniedrigung des Drucks bis auf 0,1 bis 30 mbar durchführt.

9. Verfahren nach Ansprüchen 1 bis 8, **dadurch gekennzeichnet, daß** man als zweites Lösungsmittel Benzol, Toluol, Xylol oder Chlorbenzol einsetzt.

10. Verfahren nach Ansprüchen 1 bis 9, **dadurch gekennzeichnet, daß** man Stickstoff, Luft, Sauerstoff oder ein Edelgas bei 75 bis 120°C durch das rohe Methylcarbazat leitet.

## Claims

1. Process for the preparation of methyl carbazate from hydrazine and dimethyl carbonate, **characterized in that** hydrazine and dimethyl carbonate are metered simultaneously into an initially introduced first solvent at from -20 to +30°C, the solvent and low-boiling components are then distilled off under reduced pressure, and then either a second solvent is added to the crude methyl carbazate present and this solvent is distilled off under reduced pressure or a gas is passed through the crude methyl carbazate present.

2. Process according to Claim 1, **characterized in that** hydrazine and dimethyl carbonate are employed in a molar ratio of from 0.9 to 1.1:1.

3. Process according to Claims 1 and 2, **characterized in that** the first solvent employed is water or an organic solvent which is a straight-chain, branched or cyclic, unsubstituted or substituted aliphatic compound having 1 to 20 carbon atoms, an unsubstituted or substituted aromatic compound having 6 to 10 carbon atoms, hydrazine or dimethyl carbonate, the latter two within the amount limitation quoted in Claim 5.

4. Process according to Claims 1 to 3, **characterized in that** the first solvent employed is a straight-chain or branched aliphatic alcohol having 1 to 8 carbon atoms.

5. Process according to Claims 1 to 4, **characterized in that** the simultaneous metering is carried out in such a way that one of the reactants is metered in in a molar excess of not greater than 20% (based on the other reactant) per time unit.

6. Process according to Claims 1 to 5, **characterized in that** a post-reaction time during which stirring is carried out at from +30 to +60°C for from 30 to 300 minutes is observed after the metering.

7. Process according to Claims 1 to 6, **characterized in that** the second solvent employed is an organic solvent which is a straight-chain, branched or cyclic, unsubstituted or substituted, aliphatic compound having 1 to 20 carbon atoms, an unsubstituted or substituted aromatic compound having 6 to 10 carbon atoms, hydrazine or dimethyl carbonate.

8. Process according to Claims 1 to 7, **characterized in that** the distillation is carried out with a reduction in the pressure to from 0.1 to 30 mbar.

9. Process according to Claims 1 to 8, **characterized in that** the second solvent employed is benzene, toluene, xylene or chlorobenzene.

10. Process according to Claims 1 to 9, **characterized in that** nitrogen, air, oxygen or a noble gas is passed through the crude methyl carbazate at from 75 to 120°C.

## Revendications

1. Procédé pour la préparation. du carbazate de méthyle à partir de l'hydrazine et du carbonate de diméthyle, **caractérisé en ce que** l'on introduit l'hydrazine et le carbonate de diméthyle simultanément à des températures de -20 à +30°C dans un premier solvant, on distille ensuite le solvant et les fractions volatiles sous vide puis, sur le carbazate de méthyle brut ainsi obtenu, on procède soit à l'addition d'un deuxième solvant qu'on distille sous vide, soit à l'injection d'un gaz.

2. Procédé selon la revendication 1, **caractérisé en ce que** l'on met en oeuvre l'hydrazine et le carbonate de diméthyle à un rapport molaire de 0,9 à 1,1:1.

3. Procédé selon les revendications 1 et 2, **caractérisé en ce que** le premier solvant consiste en eau ou en un solvant organique, lequel consiste en hydrocarbures aliphatiques à chaîne droite, ramifiée ou cyclique, substitués ou non en C₁-C₂₀, en hydrocarbures aromatiques substitués ou non en C₆-C₁₀, en l'hydrazine ou en le carbonate de méthyle, ces deux derniers en respectant les limitations de quantité données dans la revendication 5.

4. Procédé selon les revendications 1 à 3, **caractérisé en ce que** le premier solvant consiste en un alcool aliphatique à chaîne droite ou ramifiée en C₁-C₈.

5. Procédé selon les revendications 1 à 4, **caractérisé en ce que** l'on procède à l'introduction simultanée en respectant la règle selon laquelle, par unité de temps, on introduit l'un des réactifs en excès molaire ne dépassant pas 20 % par rapport à l'autre réactif.

6. Procédé selon les revendications 1 à 5, **caractérisé en ce que**, après l'introduction des réactifs, on observe un temps de réaction complémentaire au cours duquel on agite pendant 30 à 300 min à des températures de +30 à +60°C.

7. Procédé selon les revendications 1 à 6, **caractérisé en ce que** le deuxième solvant consiste en un solvant organique, plus spécialement un hydrocarbure aliphatique à chaîne droite, ramifiée ou cyclique, substitué ou non, en C₁-C₂₀, un hydrocarbure aromatique substitué ou non en C₆-C₁₀, l'hydrazine ou le carbonate de diméthyle.

8. Procédé selon les revendications 1 à 7, **caractérisé en ce que** la distillation est réalisée avec abaissement de la pression jusqu'à 0,1 à 30 mbar.

9. Procédé selon les revendications 1 à 8, **caractérisé en ce que** le deuxième solvant consiste en benzène, toluène, xylène ou chlorobenzène.

10. Procédé selon les revendications 1 à 9, **caractérisé en ce que** le gaz injecté dans le carbazate de méthyle brut est l'azote, l'air, l'oxygène ou un gaz rare, l'injection étant réalisée à des températures de 75 à 120°C.
